# FASCICULE DE BREVET EUROPEEN

(11) **EP 3 592 420 B1**
(45) Date de publication et mention de la délivrance du brevet: **29.06.2022**
(21) Numéro de dépôt: 18726098.9
(22) Date de dépôt: 15.05.2018
(51) Int. Cl.: A61N 1/37, A61N 1/378, A61N 1/372, A61N 1/375

(54) **IMPLANT CARDIAQUE AUTONOME DE TYPE CAPSULE LEADLESS, COMPRENANT UN RECUPERATEUR D'ENERGIE DELIVRANT DES INFORMATIONS PHYSIOLOGIQUES OU D'ACTIVITE DU PATIENT**
AUTONOMES HERZIMPLANTAT VOM TYP EINER LEITUNGSLOSEN KAPSEL MIT ENERGIERÜCKGEWINNUNG ZUR BEREITSTELLUNG VON INFORMATIONEN HINSICHTLICH DER PHYSIOLOGIE ODER AKTIVITÄT DES PATIENTEN
LEADLESS CAPSULE TYPE AUTONOMOUS CARDIAC IMPLANT COMPRISING AN ENERGY RECOVERY PROVIDING INFORMATION ON THE PHYSIOLOGY OR ACTIVITY OF THE PATIENT

(30) Priorité: 28.06.2017 FR 1755938
(43) Date de publication de la demande: 15.01.2020
(73) Titulaire: Cairdac, 92160 Antony (FR)
(72) Inventeur: BONNET, Jean-Luc, 91300 Massy (FR); FERIN, Guillaume, 37038 Tours Cedex 1 (FR)
(74) Mandataire: Dupuis-Latour, Dominique
(86) Numéro de dépôt international: PCT/EP2018/062485
(87) Numéro de publication internationale: WO 2019/001829

(56) Documents cités:
- EP-A1- 2 189 182
- EP-A1- 2 495 013
- EP-A1- 3 015 132
- EP-A1- 3 025 758
- US-A1- 2009 171 408

## Description

L'invention concerne les dispositifs médicaux implantables actifs (DMIA), en particulier les implants cardiaques chargés de surveiller l'activité du myocarde et de délivrer des impulsions de stimulation, de resynchronisation ou de défibrillation en cas de trouble du rythme détecté par le dispositif.

L'invention concerne plus précisément ceux de ces dispositifs qui incorporent un système d'auto-alimentation comprenant un récupérateur d'énergie mécanique associé à une batterie-tampon intégrée. Le récupérateur, également dénommé *harvester* ou *scavenger,* recueille l'énergie mécanique résultant des mouvements divers subis par le corps du dispositif implanté. Ces mouvements peuvent avoir pour origine un certain nombre de phénomènes se produisant au rythme des battements cardiaques, notamment les secousses périodiques de la paroi sur laquelle est ancré l'implant, les vibrations des tissus cardiaques liées entre autres aux fermetures et ouvertures des valves cardiaques, ou encore les variations de débit du flux sanguin dans le milieu environnant, qui sollicitent l'implant et le font osciller au rythme des variations de débit. L'énergie mécanique recueillie par le récupérateur est convertie en énergie électrique (tension ou courant) au moyen d'un transducteur mécano-électrique approprié, pour l'alimentation des divers circuits et capteurs du dispositif et la recharge de la batterie-tampon.

Cette technique de récupération d'énergie est particulièrement bien adaptée à l'alimentation des capsules autonomes implantées dépourvues de toute liaison physique à un dispositif distant. Ces capsules sont dénommées pour cette raison "capsules *leadless",* pour les distinguer des électrodes ou capteurs disposés à l'extrémité distale d'une sonde (*lead*) parcourue sur toute sa longueur par un ou plusieurs conducteurs reliés à un générateur connecté à l'extrémité opposée, proximale.

Dans le cas de l'application cardiaque, il peut s'agir de capsules *leadless* épicardiques, fixées à la paroi extérieure du cœur, ou bien de capsules endocavitaires, fixées à la paroi intérieure d'une cavité ventriculaire ou auriculaire, ou bien encore de capsules fixées sur une paroi d'un vaisseau à proximité du myocarde. L'invention n'est toutefois pas limitée à un type particulier de capsule, et elle est applicable indifféremment à tout type de capsule *leadless,* quelle que soit sa destination fonctionnelle.

La fixation de la capsule au site d'implantation est assurée par un système d'ancrage saillant prolongeant le corps de la capsule et destinée à pénétrer dans le tissu cardiaque, notamment au moyen d'une vis. La capsule comprend par ailleurs divers circuits électroniques, capteurs, etc. ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule, la paroi interne de l'oreillette, etc.

Le US 2009/0171408 A1 (Solem) décrit divers exemples de telles capsules intracardiaques *leadless.*

Dans tous les cas, le traitement des signaux au sein de la capsule et leur transmission à distance nécessite une énergie non négligeable par rapport aux ressources énergétiques que peut stocker cette capsule dans un très faible volume disponible. Or, compte tenu de son caractère autonome, la capsule ne peut faire appel qu'à ses ressources propres, d'où la nécessité d'un système d'auto-alimentation intégré comprenant un récupérateur d'énergie associé à une petite batterie tampon intégrée.

Il existe plusieurs types de récupérateurs d'énergie, basés sur des principes physiques différents : système de type mouvement de montre à remontage automatique, système à aimants mobiles, système à soufflet ou analogue récupérant les variations de pression du milieu sanguin, etc. L'invention concerne plus précisément les capsules *leadless* (ou dispositifs implantables analogues) dont le récupérateur d'énergie utilise un ensemble inertiel soumis aux sollicitations externes décrites plus haut, avec un tel ensemble inertiel. Cet ensemble inertiel peut notamment mettre en œuvre - mais de façon non limitative - un transducteur couplé à un mécanisme pendulaire comportant dans la capsule une masse mobile, dite "masse sismique" ou "masse inertielle". Cette masse inertielle est entrainée au gré des mouvements de la capsule, qui est soumise en permanence aux diverses sollicitations externes décrites plus haut. Après chacune de ces sollicitations, la masse inertielle, qui est couplée à un élément élastiquement déformable, oscille à une fréquence propre d'oscillation libre.

D'autres types d'ensembles inertiels pour récupérateur d'énergie présentent également ce phénomène d'oscillation. En tout état de cause, l'invention n'est pas limitée à un type particulier d'ensemble inertiel, et couvre non seulement les ensembles à transducteur électromécanique que ceux à transducteur piézoélectrique, électromagnétique, électrostatique ou tribologique, tous propres à délivrer un signal électrique oscillant sous l'effet d'une contrainte mécanique externe, et que l'on désignera par le terme générique de "traducteur".

La fréquence d'oscillation de l'ensemble inertiel, typiquement de l'ordre de quelques dizaines de hertz, est notablement supérieure à la fréquence des sollicitations cycliques externes qui correspondant à la fréquence des battements cardiaques (quelques hertz tout au plus). Ainsi, à chaque contraction cardiaque la masse sismique (ou autre organe mécanique fonctionnellement analogue) sera sollicitée avec une amplitude plus ou moins importante, puis le système inertiel oscillera plusieurs fois avec des amplitudes décroissantes (rebonds caractéristiques d'une oscillation périodique amortie), et enfin se stabilisera jusqu'au battement cardiaque suivant, où le cycle sollicitation/oscillations se reproduira de façon comparable.

L'énergie mécanique de l'oscillation de l'ensemble inertiel est, par exemple, convertie en énergie électrique par un transducteur mécano-électrique produisant un signal électrique oscillant. Ce signal est délivré à un circuit de gestion de l'alimentation de l'implant, qui redresse et régule le signal électrique pour délivrer en sortie une tension ou un courant continus stabilisés permettant d'assurer l'alimentation des divers circuits électroniques et capteurs de l'implant, ainsi que la recharge de la batterie-tampon.

Avantageusement, mais de façon non limitative, le transducteur mécano-électrique peut être un composant piézoélectrique sollicité de façon cyclique et alternative en flexion de manière à engendrer au sein du matériau que le constitue des charges électriques qui sont récupérées en surface du composant pour être utilisées par le système d'auto-alimentation de la capsule.

Le composant piézoélectrique peut par exemple être une lame piézoélectrique encastrée à l'une de ses extrémités et couplée à la masse inertielle à son autre extrémité, qui est libre. On pourra se référer notamment au EP 2 857 064 A1 (Sorin CRM) qui décrit un tel agencement de récupérateur d'énergie particulièrement adapté à l'alimentation d'une capsule *leadless.*

Il a été proposé, outre la récupération d'énergie électrique, d'utiliser le signal électrique délivré par le transducteur pour obtenir des informations sur l'état clinique du cœur du patient.

Ainsi, le US 2009/0171408 A1 (Solem) précité propose de dériver du signal de conversion électrique une "information d'énergie" permettant d'évaluer des paramètres tels que le rythme cardiaque, ou l'amplitude ou l'accélération des battements cardiaques. L'analyse repose sur la constatation que l'énergie électrique délivrée par le générateur, c'est-à-dire la quantité d'électricité produite sur une durée donnée, est un indicateur de l'énergie cinétique du cœur, reflétant l'accélération et les mouvements du site où est implanté le dispositif. En d'autres termes et de façon simplifiée, plus le signal électrique est fort, plus cela révèle une bonne condition du myocarde. On obtient ainsi une évaluation de la condition globale du myocarde à partir du niveau moyen de l'énergie obtenue en sortie du générateur.

De façon comparable, le US 2015/0224325 A1 (Imran) décrit la possibilité d'utiliser le signal électrique du récupérateur d'énergie également comme un signal de capteur permettant d'assurer un suivi clinique du patient. En particulier, il est possible de détecter l'apparition d'une fibrillation ou épisode analogue par une chute soudaine du niveau moyen de tension du signal électrique délivré par le récupérateur d'énergie. Une alerte peut alors être transmise à un dispositif, implanté ou non, de *monitoring* (surveillance) porté par le patient qui surveille en continu le signal d'alimentation délivré par le récupérateur.

Il s'agit toutefois, dans l'un ou l'autre cas, d'informations cliniques relativement sommaires et/ou d'évolution lente, déduites de l'amplitude moyenne du signal électrique évaluée sur plusieurs cycles cardiaques successifs.

Le but de l'invention est de proposer une technique d'analyse du signal électrique d'alimentation délivré par le récupérateur d'énergie d'un dispositif implanté autonome, qui permette :
- d'obtenir des informations cliniques beaucoup plus spécifiques que celles qu'il était possible d'obtenir avec les techniques antérieures connues ;
- notamment, d'obtenir de telles informations cliniques aussi bien de nature physiologique (portant sur le comportement intrinsèque du myocarde) que de nature physique (donnant une indication du niveau d'activité du patient à un instant donné) ; et
- d'obtenir toutes ces informations en temps réel, immédiatement après chaque cycle cardiaque.

On sait obtenir de telles informations avec un dispositif implanté, notamment un dispositif de type capsule *leadless,* mais il est pour cela nécessaire que la capsule intègre un ou plusieurs capteurs spécifiques, tels qu'un accéléromètre (capteur G) pour l'évaluation du niveau d'activité et/ou un capteur d'accélération endocardiaque (capteur EA) pour l'évaluation de paramètres tels que la contractilité du myocarde, comme cela est par exemple décrit par les EP 3 025 758 A1 ou EP 2 495 013A1 (Sorin CRM), mais dans un tout autre contexte qui est celui de l'analyse d'un signal issu d'un capteur EA. Un signal EA n'a en effet rien de comparable, ni dans sa nature ni dans sa forme, avec le signal électrique oscillant amorti produit par l'ensemble inertiel d'un récupérateur d'énergie. L'idée de base de l'invention consiste à analyser les variations à très court terme du signal électrique délivré par le récupérateur d'énergie, entre deux battements cardiaques.

Cette analyse est opérée en temps réel sur le signal reflétant les oscillations de l'ensemble inertiel (typiquement, les oscillations de la masse inertielle dans le cas d'un ensemble pendulaire) à sa fréquence d'oscillation juste après la contraction cardiaque, correspondant donc aux oscillations amorties ou "rebonds" décrits plus haut, et ceci à l'intérieur d'un même cycle cardiaque.

À la différence des techniques connues, par exemple celle décrite par le US 2009/0171408 A1 précité, il ne s'agit pas d'analyser l'évolution du niveau moyen du signal électrique d'un cycle cardiaque au suivant, ou sur plusieurs cycles cardiaques successifs, donc d'analyser des variations beaucoup plus lentes.

Comme on le verra, la technique d'analyse selon l'invention permet d'obtenir nombre d'informations cliniques importantes, aussi bien de nature physique que de nature physiologique, telles que le niveau d'activité instantané du patient, le degré de contractilité du myocarde, éventuellement en distinguant contraction isovolumique et éjection ventriculaire, etc.

Avantageusement, ces informations sont obtenues à partir du seul signal électrique produit par le récupérateur d'énergie, sans qu'il soit pour cela nécessaire de munir l'implant d'un ou plusieurs capteurs spécifiques, car l'ensemble inertiel (typiquement, l'ensemble pendulaire masse inertielle/ transducteur mécano-électrique) joue un double rôle de récupérateur d'énergie et de capteur physiologique et/ou d'activité physique. L'invention propose à cet effet un implant cardiaque autonome de type capsule *leadless* comprenant, de manière en elle-même connue d'après le US 2009/0171408 A1 précité: un corps d'implant muni de moyens d'ancrage à une paroi cardiaque, le corps d'implant logeant un ensemble électronique ; et un module de récupération d'énergie avec un organe de stockage d'énergie pour l'alimentation de l'ensemble électronique.

Le module de récupération d'énergie est apte à convertir en énergie électrique des sollicitations externes appliquées au corps de l'implant sous l'effet de mouvements d'une paroi à laquelle est ancré l'implant et/ou de variations de débit du flux sanguin dans le milieu environnant l'implant au rythme de battements cardiaques et/ou de vibrations de tissus cardiaques.

Le module de récupération d'énergie comprend : un ensemble inertiel soumis aux sollicitations externes ; un traducteur apte à convertir l'énergie mécanique produite par les oscillations de l'ensemble inertiel en un signal électrique oscillant ; et un circuit de gestion d'alimentation, apte à redresser et réguler le signal électrique oscillant pour délivrer en sortie une tension ou un courant continus stabilisés pour l'alimentation de l'ensemble électronique et/ou la recharge de l'organe de stockage d'énergie. Le dispositif comprend en outre un module d'analyse recevant en entrée le signal électrique délivré par le traducteur et apte à analyser des variations de ce signal électrique oscillant pour en déduire une valeur d'un paramètre physiologique du patient porteur de l'implant.

De façon caractéristique de l'invention : l'implant comprend en outre un module de séquencement comprenant un circuit de détection d'événements cardiaques ventriculaires ou auriculaires successifs, et un circuit de fenêtrage apte à définir au moins une fenêtre temporelle prédéterminée entre deux évènements cardiaques consécutifs détectés ; et le module d'analyse est apte à analyser les variations instantanées dudit signal électrique oscillant à l'intérieur de la(les) fenêtre(s) temporelle(s) prédéterminée(s) ainsi définie(s) entre deux évènements cardiaques consécutifs détectés, et en déduire une valeur courante i) d'au moins un paramètre physiologique, et/ou ii) d'au moins un paramètre d'activité physique, du patient porteur de l'implant.

Selon diverses caractéristiques subsidiaires avantageuses :
- l'ensemble inertiel comprend un ensemble pendulaire avec un élément élastiquement déformable suivant au moins un degré de liberté, couplé à une masse inertielle. Dans un tel cas, le module de récupération d'énergie comprend avantageusement au moins une lame piézoélectrique couplée à l'une de ses extrémités à la masse inertielle, ladite lame piézoélectrique formant à la fois ledit élément élastiquement déformable et ledit traducteur ;
- le circuit de fenêtrage est apte à définir une première fenêtre temporelle de recherche d'un premier pic du signal électrique oscillant ;
- le circuit de fenêtrage est apte à définir une deuxième fenêtre temporelle correspondant à une phase en oscillation libre amortie de l'ensemble inertiel ;
- le circuit de fenêtrage est apte à rechercher un pic d'amplitude de la première oscillation du signal électrique, et à définir la deuxième fenêtre temporelle en fonction de l'instant dudit pic d'amplitude ;
- le module d'analyse est apte à déterminer une grandeur liée à au moins une partie de la durée d'une alternance de la première oscillation du signal électrique ; et déduire au moins un paramètre physiologique du patient en fonction de ladite grandeur. Ladite grandeur est avantageusement l'une d'entre : l'amplitude d'un premier pic de signal de la première alternance du signal électrique oscillant ; la dérivée de l'amplitude du signal électrique oscillant au début de la première alternance ; ou l'intégrale de l'amplitude du signal électrique oscillant sur tout ou partie de la durée de la première alternance ;
- le module d'analyse est apte à : discriminer une première oscillation et une seconde oscillation consécutive du signal électrique survenant dans la première sous-fenêtre ; déterminer deux grandeurs liées à au moins une partie de la durée d'une alternance respective desdites première et seconde oscillations ; et déduire i) deux paramètres physiologiques distincts du patient en fonction de ces deux grandeurs respectives ou ii) un paramètre physiologique du patient en fonction d'une combinaison de ces deux grandeurs respectives ;
- le circuit de fenêtrage est apte à définir une troisième fenêtre temporelle, postérieure à la deuxième fenêtre temporelle, correspondant à une phase substantiellement non oscillante du système inertiel en oscillation libre amortie ;
- le module d'analyse est apte à : déterminer une grandeur liée aux variations du signal électrique pendant la troisième fenêtre temporelle ; et déduire au moins un paramètre d'activité physique du patient en fonction de ladite grandeur ;
- ladite grandeur est l'énergie du signal électrique redressé et intégré pendant la durée de la seconde sous-fenêtre.

On va maintenant décrire un exemple de mise en œuvre de la présente invention, en référence aux dessins annexés où les mêmes références désignent d'une figure à l'autre des éléments identiques ou fonctionnellement semblables.
La Figure 1 illustre un dispositif médical de type capsule *leadless* dans son environnement, avec divers exemples de sites d'implantation dans, sur ou à proximité du cœur d'un patient.
La Figure 2 est une vue de côté générale d'une capsule *leadless* comprenant un récupérateur d'énergie à ensemble pendulaire.
La Figure 3 montre, isolément, l'ensemble pendulaire de la capsule *leadless* de la Figure 2, avec une lame piézoélectrique couplée à une masse inertielle.
La Figure 4 est un chronogramme des oscillations du signal électrique délivré par le module de récupération d'énergie de la capsule *leadless* au cours de cycles cardiaques successifs, avec en regard le tracé d'électro-gramme correspondant.
La Figure 5 présente, sous forme de schéma par blocs, les principaux éléments constitutifs internes de l'ensemble électronique de la capsule *leadless.*
La Figure 6 est un chronogramme des oscillations du signal électrique, illustrant notamment les caractéristiques de la première oscillation à analyser afin d'en dériver un ou plusieurs paramètres physiologiques propres au patient porteur de la capsule *leadless.*
La Figure 7 est un chronogramme des oscillations du signal électrique, illustrant la possibilité de discriminer les deux phases de contraction isovolumique et d'éjection ventriculaire d'un même cycle cardiaque, pour permettre une analyse spécifique de la contractilité cardiaque sur ces deux phases.
Les Figures 8a à 8c sont des chronogrammes des oscillations du signal électrique, illustrant la manière d'analyser ce signal après la phase de rebonds de manière à en dériver un paramètre d'activité physique instantanée du patient porteur de la capsule *leadless.*
La Figure 9 est un organigramme détaillant, selon un exemple illustratif de mise en œuvre de l'invention, la séquence d'opérations et de tests effectués sur le signal électrique délivré par le module de récupération d'énergie, en fonction des évènements cardiaques détectés, pour l'obtention de données permettant de déterminer des paramètres physiologiques et d'activité physique.

On va maintenant décrire un exemple de réalisation du dispositif de l'invention.

En ce qui concerne ses aspects logiciels, l'invention peut être mise en œuvre par une programmation appropriée du logiciel de commande d'un dispositif stimulateur cardiaque connu, par exemple un stimulateur de type capsule *leadless* endocavitaire.

Ces dispositifs comprennent un microprocesseur programmable couplé à des circuits pour recevoir, mettre en forme et traiter des signaux électriques recueillis par des électrodes implantées, et délivrer des impulsions de stimulation à ces électrodes. L'adaptation de ces dispositifs pour réaliser l'invention est à la portée de l'homme du métier, et elle ne sera pas décrite en détail. En particulier, les logiciels conservés en mémoire et exécutés pourront être adaptés et utilisés pour mettre en œuvre les fonctions de l'invention qui seront décrites ci-dessous

Le procédé de l'invention est en effet mis en œuvre par des moyens principalement logiciels, au moyen d'algorithmes appropriés exécutés par un microcontrôleur ou un processeur numérique de signal. Pour la clarté de l'exposé, les divers traitements appliqués seront décomposés et schématisés par un certain nombre de modules ou blocs fonctionnels distincts et/ou de circuits interconnectés, mais cette représentation n'a toutefois qu'un caractère illustratif, ces fonctions ou circuits comprenant des éléments communs et correspondant en pratique à une pluralité de fonctions globalement exécutées par un même logiciel.

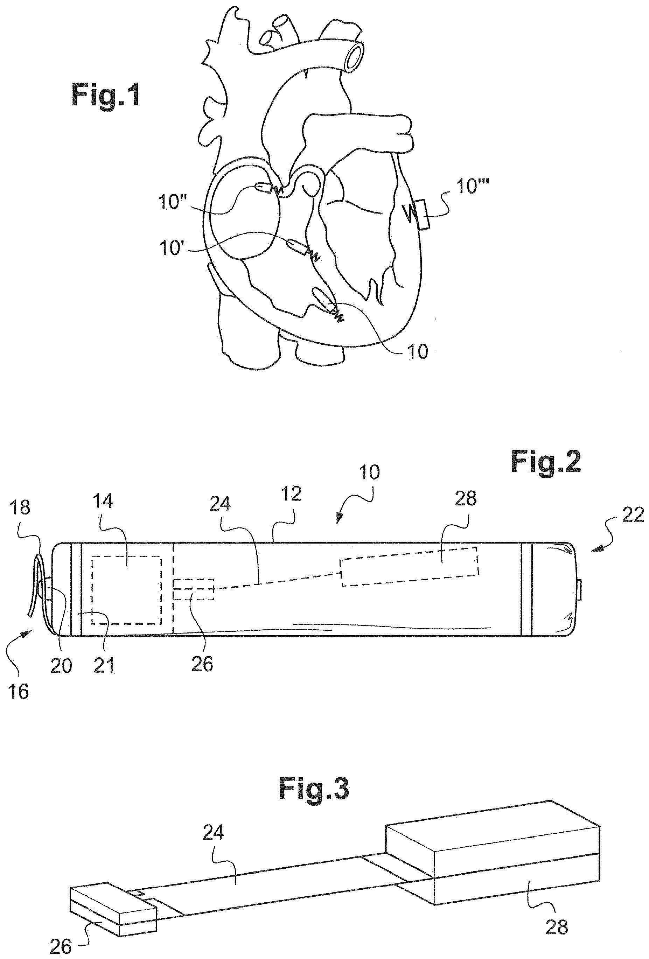

Sur la Figure 1, on a représenté diverses possibilités de site d'implantation d'un dispositif de type *leadless,* à l'intérieur du myocarde (implant endocavitaire) ou sur une région externe de ce même myocarde (implant épicardique), ou encore sur ou dans un vaisseau proche du cœur. Dans un exemple préférentiel avantageux, la capsule *leadless* 10 est implantée à l'apex du ventricule droit. En variante, la capsule peut être également implantée dans le ventricule droit sur le septum interventriculaire, ou encore sur une paroi de la cavité auriculaire droite, comme illustré respectivement en 10' et 10". Une autre configuration consiste, comme en 10‴, à implanter la capsule *leadless* sur une paroi externe du myocarde. Dans tous les cas, la capsule *leadless* est fixée à la paroi cardiaque au moyen d'une vis d'ancrage saillante destinée à pénétrer dans le tissu cardiaque par vissage au site d'implantation. D'autres systèmes d'ancrage sont utilisables, et ne modifient en aucune façon la mise en œuvre de la présente invention.

La Figure 2 est une vue de côté générale d'une telle capsule *leadless,* comprenant un récupérateur d'énergie à ensemble pendulaire.

La capsule *leadless* 10 est dans cet exemple réalisée sous forme extérieure d'un corps d'implant tubulaire cylindrique 12 enfermant un ensemble 14 incluant les divers circuits électroniques et d'alimentation de la capsule. Les dimensions typiques d'une telle capsule sont un diamètre de l'ordre de 6 mm environ pour une longueur d'environ 25 mm.

À son extrémité distale 16, la capsule porte une vis hélicoïdale 18 pour l'ancrage de la capsule contre une paroi d'une cavité cardiaque, comme illustré ci-dessus à propos de la Figure 1. Une électrode de détection/stimulation 20, en contact avec le tissu cardiaque au site d'implantation, assure le recueil des potentiels de dépolarisation cardiaque et/ou l'application d'impulsions de stimulation. Dans certaines formes de réalisation, la fonction de l'électrode 20 est assurée par la vis d'ancrage 18, qui est alors une vis active, électriquement conductrice et reliée aux circuits de détection/stimulation de la capsule. L'électrode 20 en contact avec les tissus est généralement une cathode, et elle est associée à une anode dont la fonction est assurée par une seconde électrode distante, le plus souvent une électrode annulaire telle qu'en 21.

L'extrémité proximale opposée 22 de la capsule *leadless* 10 présente une forme arrondie atraumatique, et elle est pourvue de moyens appropriés de préhension pour la liaison à un cathéter-guide ou autre accessoire d'implantation utilisable au moment de la mise en place ou de l'explantation de la capsule.

La capsule *leadless* 10 est pourvue d'un module de récupération d'énergie destiné à alimenter l'ensemble électronique 14 et/ou à recharger une batterie intégrée ou un condensateur de stockage d'énergie.

Un tel module de récupération d'énergie comprend un ensemble inertiel qui, à l'intérieur de la capsule, oscille au gré des diverses sollicitations externes auxquelles la capsule *leadless* est soumise. Ces sollicitations peuvent notamment résulter : des mouvements de la paroi à laquelle est ancrée la capsule, qui sont transmis au corps d'implant 12 par la vis d'ancrage 18; et/ou des variations du début du flux sanguin dans le milieu environnant l'implant, qui produisent des oscillations du corps d'implant au rythme des battements cardiaques ; et/ou des vibrations diverses transmises par les tissus cardiaques.

La Figure 3 illustre un exemple d'ensemble inertiel pour module de récupération d'énergie, ensemble qui est ici constitué d'une lame piézoélectrique 24 encastrée à l'une de ses extrémités 26 et dont l'extrémité opposée, libre, est couplée à une masse inertielle 28. La lame piézoélectrique 24 est une lame flexible qui, dans l'exemple illustré, est élastiquement déformable suivant au moins un degré de liberté en flexion longitudinale.

Du point de vue mécanique cet ensemble constitue un ensemble pendulaire de type masse-ressort (le ressort étant constitué par la lame piézoélectrique flexible) qui, du fait de l'inertie de la masse 28, oscille dès que la lame élastique 24 est écartée de sa position stable de repos. De fait, cet ensemble peut être assimilé, pour ce qui est de son comportement mécanique, à une structure de type "poutre encastrée-libre", qui présente une fréquence propre d'oscillation libre, qui est ici la fréquence sur laquelle oscille le système masse-ressort.

La lame piézoélectrique 24 assure en outre une fonction de transducteur mécano-électrique permettant de convertir en charges électriques la sollicitation mécanique qui lui est appliquée lorsqu'elle est fléchie, charges récupérées par des électrodes formées à la surface de la lame. La lame est de préférence une lame de type bimorphe, c'est-à-dire capable de générer de l'énergie sur ses deux faces lorsqu'elle est soumise à une déformation. Ces propriétés de transduction sont typiques d'un matériau piézoélectrique tel que les céramiques PZT ou les monocristaux de type PMN-PT, titanate de baryum ou niobate de lithium.

Bien entendu, l'invention n'est pas limitée à cette configuration particulière d'ensemble pendulaire donnée en exemple, et d'autres types d'ensembles inertiels peuvent être utilisés pour la récupération d'énergie, tels que ceux évoqués en introduction, dès lors qu'ils présentent au moins une fréquence propre d'oscillation libre.

De même, l'ensemble inertiel peut être un ensemble déformable suivant plusieurs degrés de liberté, avec alors autant de modes vibratoires correspondants et de fréquences propres d'oscillation, et de signaux électriques respectivement délivrés en sortie.

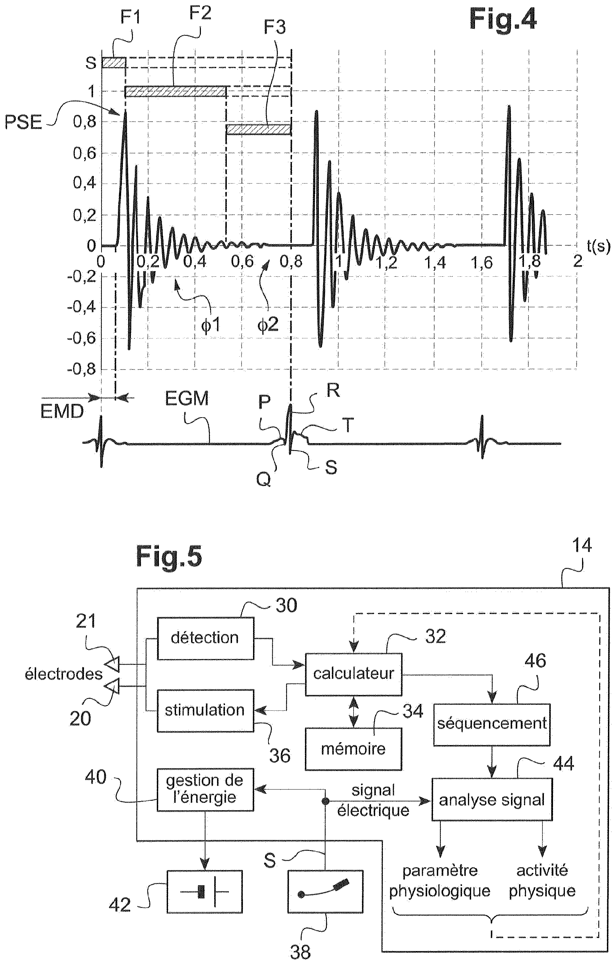

La Figure 4 est un chronogramme des oscillations du signal électrique S délivré par le module de récupération d'énergie de la capsule *leadless* au cours de cycles cardiaques successifs, avec en regard le tracé d'électrogramme correspondant.

Le signal d'électrogramme EGM, qui est le signal électrique de l'onde de dépolarisation cardiaque, est par exemple détecté par l'électrode 20 en contact avec le myocarde. Sur l'exemple illustré Figure 4, l'électrogramme est celui d'un rythme sinusal, avec une onde P correspondant à l'activité électrique auriculaire, un complexe QRS correspondant à l'activité électrique ventriculaire et une onde T correspondant à la repolarisation ventriculaire. Des moyens de filtrage et de traitement du signal, inclus dans les circuits de l'ensemble 14, permettent de dériver aisément de l'EGM un marqueur temporel R indiquant l'instant de la dépolarisation ventriculaire. Si la dépolarisation résulte d'une stimulation électrique délivrée par la capsule *leadless,* le marqueur correspondant V correspond à l'instant de délivrance de l'impulsion. Il est également possible d'obtenir de la même façon des marqueurs de l'activité électrique auriculaire P (spontanée) ou A (stimulée).

Dans la suite, on fera principalement référence à un marqueur R (dépolarisation ventriculaire spontanée), mais il conviendra d'assimiler à ce cas celui d'un marqueur V (dépolarisation ventriculaire stimulée) ou d'un marqueur P/A (dépolarisation auriculaire spontanée ou stimulée).

Ce mode de détection des évènements cardiaques (événements ventriculaires ou évènements auriculaires) à partir d'un signal EGM recueilli par un système d'électrodes n'est toutefois pas limitatif, et il existe d'autres techniques de détection, notamment des techniques de détection de nature mécanique (non électriques) telles que celles obtenues par analyse du signal délivré par un accéléromètre intégré à l'implant, cet accéléromètre délivrant un signal représentatif de l'accélération endocardiaque (EA). Le EP 3 025 758 A1 décrit une telle technique, où la capsule *leadless* comprend un capteur EA dont le signal est analysé pour détecter la capture ou l'absence de capture (présence ou non d'une contraction du ventricule) suite à l'application d'une impulsion de stimulation. Toutefois, dans une telle technique connue, ces signaux ne sont plus reliés à l'activité électrique du myocarde, mais à son activité mécanique.

La Figure 4 illustre également, en regard de l'électrogramme EGM, les variations du signal électrique S délivré par le récupérateur d'énergie, c'est-à-dire le signal variable produit par le transducteur mécano-électrique constitué par la lame piézoélectrique 24, dans l'exemple illustré.

Ce signal est un signal récurrent, se répétant au rythme des battements cardiaques successifs, et il comporte à chaque occurrence deux phases caractéristiques qui se succèdent, Ø1 et Ø2.

La première phase Ø1 est constituée d'une suite d'oscillations sinusoïdales amorties, avec un premier pic d'amplitude suivi d'une série de "rebonds" d'amplitudes décroissantes. La première oscillation de cette phase Ø1 survient après un délai EMD dit "délai électromécanique" correspondant au retard physiologique entre i) l'arrivée de l'onde de dépolarisation (spontanée ou stimulée) au niveau des tissus du ventricule, et ii) la contraction mécanique du myocarde produite par cette onde de dépolarisation.

La première phase Ø1 d'oscillations amorties est suivie d'une seconde phase, consécutive, Ø2 substantiellement sans rebonds, qui se prolonge jusqu'à une nouvelle contraction du myocarde produisant des variations similaires du signal S.

L'ordre de grandeur de la fréquence de récurrence des cycles cardiaques est typiquement de 1 à 2 Hz (60 à 120 bpm (battements par minute)). La fréquence propre de l'ensemble pendulaire, quant à elle, est déterminée par la géométrie de la lame piézoélectrique 24 (principalement sa longueur et son épaisseur), par l'élasticité du matériau qui la compose, et par la masse de la masse inertielle 28. Ces différents paramètres sont choisis de manière à donner à la fréquence propre d'oscillation libre une valeur beaucoup plus élevée que la fréquence du rythme cardiaque, par exemple une fréquence de l'ordre de 20 Hz, ce chiffre n'étant bien entendu aucunement limitatif. Il s'agit que, pratiquement en toutes circonstances, l'ensemble pendulaire produise entre deux battements cardiaques une pluralité de rebonds suivis par une phase sans rebonds avant le battement cardiaque suivant.

La Figure 5 est un synoptique de l'ensemble électronique 14 intégré à la capsule *leadless* 10, présenté sous forme de blocs fonctionnels. Ce circuit 14 est avantageusement implémenté sous forme d'un ASIC ou d'une combinaison d'ASICs.

Le bloc 30 désigne un circuit de détection de l'onde de dépolarisation cardiaque, relié à l'électrode 20 en contact avec le tissu cardiaque et à l'électrode opposée 21. Le bloc 30 comprend des filtres et des moyens de traitement analogique et/ou numérique du signal recueilli. Le signal ainsi traité est appliqué à l'entrée d'un calculateur 32 associé à une mémoire 34. Le calculateur 32 opère à partir des signaux recueillis une détection des évènements cardiaques ventriculaires ou auriculaires successifs, de manière à générer (entre autres) une série de marqueurs R/V (et/ou P/A) successifs au rythme des battements cardiaques.

L'ensemble électronique 14 comporte également un circuit de stimulation 36 opérant sous le contrôle du calculateur 32 pour, en tant que de besoin, délivrer au système d'électrodes 20 et 21 des impulsions de stimulation du myocarde et générer des marqueurs V ou A correspondants

Il est par ailleurs prévu un circuit de récupération d'énergie 38, constitué par l'ensemble pendulaire formé par la lame piézoélectrique 24 et la masse inertielle 28 décrits plus haut en référence aux Figures 2 et 3.

Ce circuit de récupération d'énergie 38 produit en sortie un signal électrique variable S, dont les variations sont illustrées dans diverses situations notamment sur la Figure 4 et les Figures 6 à 8.

Le signal S est, en premier lieu, délivré à un circuit 40 de gestion de l'alimentation, qui redresse et régule le signal S de manière à produire en sortie une tension ou un courant continus stabilisés servant à l'alimentation de l'ensemble électronique 14 et à la recharge d'une batterie tampon 42 ou d'un dispositif analogue tel qu'un condensateur de stockage d'énergie.

Ce même signal S est, en second lieu, appliqué à un circuit 44 d'analyse des variations instantanées du signal afin de délivrer en sortie, de façon caractéristique et de la manière qui sera décrite plus bas, au moins un paramètre physiologique du patient porteur de l'implant et/ou au moins un paramètre d'activité physique de ce même patient (ou, à tout le moins, des données d'état permettant de dériver ces paramètres). Ces paramètres pourront notamment être utilisés par le calculateur 32 pour assurer un suivi de l'état du patient à court terme, moyen terme ou long terme et moduler en tant que de besoin l'application des impulsions de stimulation par le circuit 36.

Le circuit d'analyse 44 opère sous le contrôle d'un circuit de séquencement 46 qui définit la position temporelle d'une ou plusieurs fenêtres d'analyse sur la base des évènements électriques cardiaques déterminés (R/V et/ou P/A) par le calculateur 32, ces fenêtres bornant les divers traitements opérés par le circuit 44 en vue d'extraire du signal brut S les informations significatives recherchées.

Avantageusement, comme illustré Figure 4, il est prévu trois fenêtres désignées F1, F2 et F3, successivement déclenchées par le circuit de séquencement 46.

La première fenêtre F1 est une fenêtre de scrutation du signal S, déclenchée sur détection d'un évènement cardiaque (événement ventriculaire dans l'exemple illustré) spontané ou stimulé.

La fonction de cette fenêtre F1 est de commencer à suivre et analyser les variations du signal électrique S, de manière à y détecter la survenue du premier maximum de l'oscillation libre de l'ensemble pendulaire (lame piézoélectrique 24 et masse inertielle 28). Ce premier maximum est le pic de signal électrique PSE sur la Figure 4.

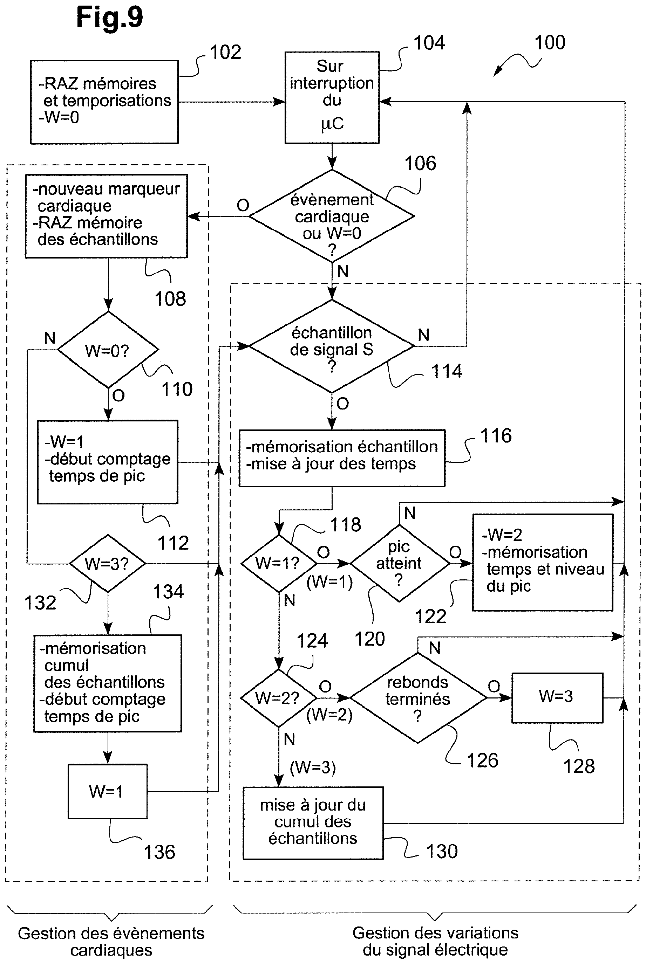

Le détail de la recherche du pic PSE, ainsi que des étapes qui lui font suite, sera donné plus bas en référence à la Figure 9.

D'autres critères de déclenchement de la fenêtre F1 de scrutation du signal sont envisageables, en variante ou en complément d'une simple détection du pic PSE du signal électrique, notamment :
- sur détection de l'événement cardiaque, l'application d'un délai fixe ou paramétrable avant déclenchement de la fenêtre F1, notamment pour tenir compte de l'écoulement du délai électromécanique EMD : une variation du signal qui serait détectée dans cet intervalle correspondrait en effet à un artefact, à ignorer dans la recherche du pic d'oscillation ; ou
- l'application d'un délai semblable, mais variable cycle-à-cycle en fonction du rythme cardiaque, par exemple un délai calculé comme étant un pourcentage de l'intervalle RR (ou W, RV ou VR) précédent.

### Obtention d'un paramètre physiologique du patient

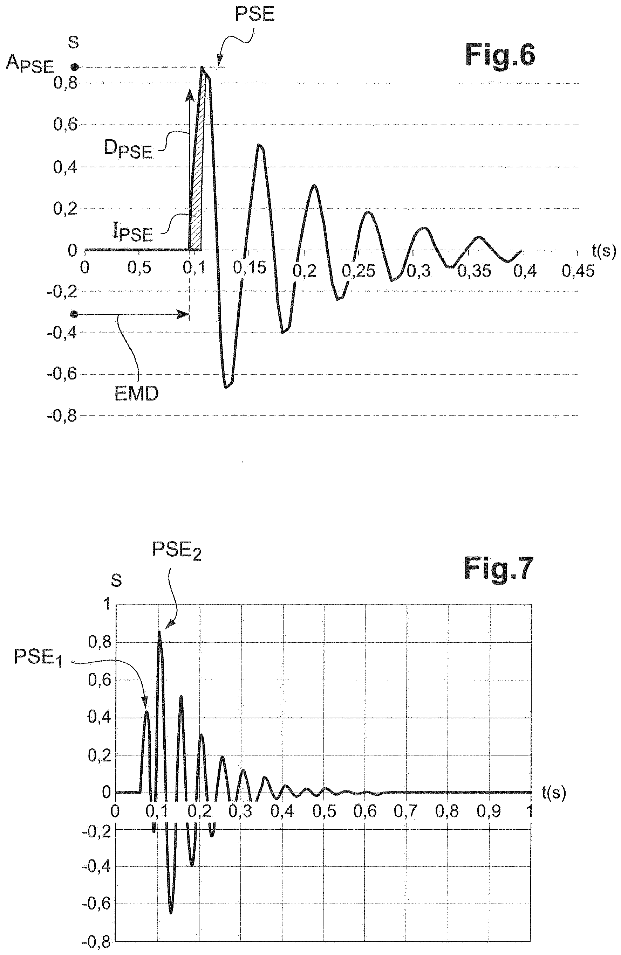

La Figure 6 illustre, plus précisément, diverses manières possibles de dériver une information représentative d'un paramètre physiologique du patient une fois détecté le pic de signal électrique PSE, notamment à partir :
- de l'amplitude maximale A_{PSE} du signal électrique S (c'est-à-dire du niveau d'amplitude du pic PSE) ;
- d'une dérivation de l'amplitude du signal électrique S au début de la première alternance (celle du pic PSE), comme illustré sur la Figure 4 par la pente D_{PSE} du signal S ;
- d'une intégration de l'amplitude du signal électrique S sur tout ou partie de la durée de la première alternance, par exemple, comme illustré Figure 4, sur la partie I_{PSE} de la première demi-alternance qui est comprise entre l'instant du début de l'alternance et l'instant du pic PSE.

Chacune de ces données, ou une combinaison de ces données, permet de dériver un paramètre physiologique du patient, représentatif notamment de la contractilité du myocarde.

En complément, la durée écoulée entre l'instant de détection de l'événement cardiaque et l'instant de début de la première oscillation du signal, qui est une durée correspondant au délai électromécanique EMD, peut également fournir une information pertinente sur la contractilité du myocarde.

Le(s) paramètre(s) physiologique(s) ainsi déterminé(s) peu(ven)t être mémorisé(s) dans une mémoire du dispositif à des fins de diagnostic, suivant des techniques d'analyse en elles-mêmes connues et qui ne font pas partie de l'invention, notamment des techniques :
- d'analyse des variations à court terme, cycle-à-cycle, du (des) paramètre(s), permettant notamment de détecter une situation de début d'infarctus ;
- d'analyse des variations à moyen terme, de l'ordre de quelques jours, du (des) paramètre(s), permettant notamment de diagnostiquer des pathologies à évolution lente telles qu'ischémie ou décompensation cardiaque ;
- d'analyse des variations à long terme, de l'ordre de quelques semaines ou quelques mois, du (des) paramètre(s), permettant d'évaluer des pathologies à évolution très lente, notamment dans le cadre de la prévention ou du suivi de l'insuffisance cardiaque.

La Figure 7 illustre une autre possibilité offerte par la technique de l'invention, qui permet de discriminer dans certaines configurations les composantes principales successives de la contraction myocardique, à savoir i) la contraction isovolumique et ii) l'éjection ventriculaire immédiatement consécutive.

Cette discrimination est opérée par analyse des deux premiers pics du signal électrique S : si l'on détecte sur ce signal, comme illustré Figure 7, un premier pic mineur PSE1 immédiatement suivi d'un second pic majeur PSE2, d'amplitude supérieure, alors ceci signifie que le premier pic PSE1 est celui produit par la contraction isovolumique et le second pic PSE2 est celui produit par l'éjection ventriculaire (on notera que ces deux pics, qui sont distinguables dans l'exemple de la Figure 7, sont en revanche confondus en un même pic PSE sur les exemples de chronogrammes des Figures 4 et 6).

Une fois déterminés les instants de survenue et les amplitudes de chacun des pics PSE1 et PSE2, le dispositif peut dériver de ces informations un ou plusieurs paramètres physiologiques et/ou d'activité physique du patient, à partir notamment :
- de l'amplitude de crête du premier pic PSE1 (valeur liée notamment au niveau d'activité du patient) ;
- de l'instant d'apparition du premier pic PSE1 ;
- de l'intervalle de temps séparant les deux pics PSE1 et PSE2.

L'analyse combinée d'informations représentatives respectivement de la contraction isovolumique et de l'éjection ventriculaire est une technique en elle-même connue, par exemple d'après les EP 2 495 013 A1 et EP 2 684 515 A1, où ces informations sont obtenues à partir d'un signal d'accélération endocardiaque (EA), qui est un signal d'origine mécanique délivré par un capteur accélérométrique disposé par exemple en bout d'une sonde endocavitaire implantée au fond du ventricule.

Dans tous les cas et de façon générale, en variante ou en complément de l'utilisation à des fins de diagnostic du (des) paramètre(s) physiologique(s) obtenu(s) par la technique de l'invention, ce(s) paramètre(s) peu(ven)t être utilisé(s) à des fins d'asservissement de la fréquence de stimulation cardiaque.

En effet, la contractilité cardiaque augmentant avec l'effort, ce paramètre physiologique peut être utilisé pour contrôler le rythme de délivrance des impulsions de stimulation, dans le cas où le patient n'est pas en rythme sinusal mais est stimulé par le dispositif implanté.

### Obtention d'un paramètre d'activité physique du patient

On va maintenant décrire la fonction des deux fenêtres suivantes F2 et F3.

La détection du pic PSE déclenche une deuxième fenêtre F2 (Figure 4), qui est une fenêtre d'effacement de la phase de rebonds.

La fonction de cette fenêtre F2 est d'analyser les variations du signal S de manière à borner la phase Ø1 des rebonds successifs (oscillations amorties) de l'ensemble pendulaire, pour déterminer un instant où l'on pourra considérer que cette phase Ø1 de rebonds est terminée.

La fin de la phase de rebonds peut être définie selon plusieurs critères possibles, éventuellement combinables entre eux, tels que :
- durée, fixe ou paramétrable, comptée à partir de la détection du premier pic PSE du signal S ;
- durée variable en fonction du rythme cardiaque, par exemple une durée de 50 % de l'intervalle RR précédent, ou d'une moyenne des intervalles RR précédents ;
- amplitude du signal S devenant, sur au moins une oscillation complète, inférieure à un seuil donné, qui peut être un seuil fixe ou paramétrable ou bien un seuil variable correspondant à un pourcentage donné (par exemple 5 %) de l'amplitude du pic PSE ;
- variabilité du signal S devenant inférieure à un seuil donné : en effet, du fait de l'amortissement de la sinusoïde cette variabilité diminue au fur et à mesure des oscillations successives.

La détermination de la fin de la phase de rebonds (fin de la phase Ø1) a pour effet de déclencher une troisième fenêtre F3, qui est une fenêtre de détection d'activité du patient.

Cette fenêtre F3 s'étend sur une durée (phase Ø2) comprise entre la fin de la sinusoïde amortie et le début de la contraction cardiaque suivante. Dans la mesure où l'ensemble pendulaire a achevé sa phase d'oscillation libre consécutive à la contraction cardiaque (phase Ø1), les variations du signal qui peuvent être détectées dans tout ou partie de cette fenêtre F3 ne proviennent pas de la contraction cardiaque, mais de sollicitations externes au myocarde. Concrètement, ces sollicitations résultent essentiellement des mouvements du patient, c'est-à-dire de son niveau d'activité physique propre : marche, montée d'escalier, exercice, mouvements divers du buste, etc., qui induisent des mouvements irréguliers de la masse inertielle, donc des déformations de la lame piézoélectrique et par voie de conséquence la production de charges électriques et d'une tension ou d'un courant correspondants en sortie du récupérateur d'énergie.

L'évaluation de ce niveau d'activité peut notamment être obtenue par redressement et intégration du signal S sur tout ou partie de la durée de la fenêtre F3.

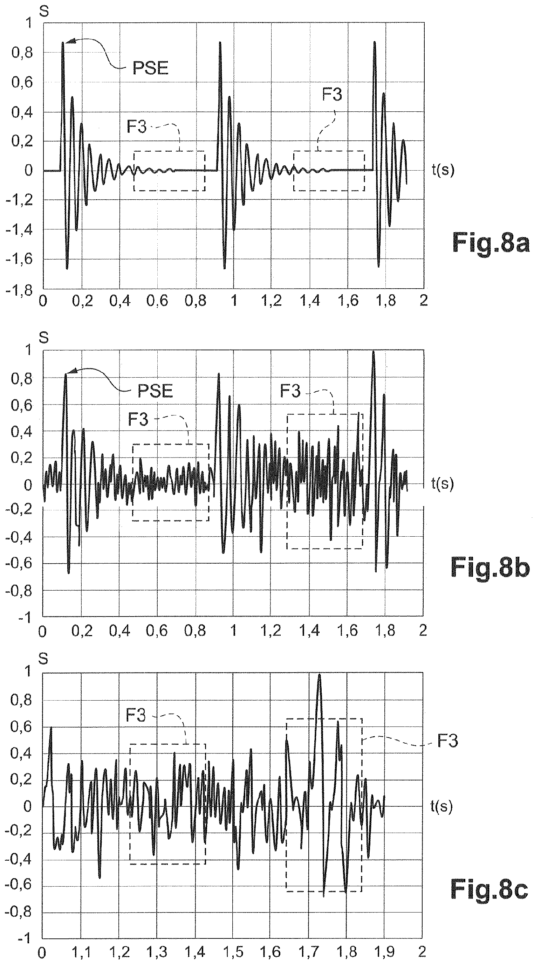

Les Figures 8a à 8c illustrent des exemples de variation du signal électrique S dans diverses situations d'activité du patient.

La Figure 8a est un exemple de signal recueilli lorsque le patient est au repos. La phase de rebonds et suivie par une phase, indiquée en tiretés et correspondant à la fenêtre F3, durant laquelle les variations du signal S sont très faibles, voire imperceptibles, jusqu'à la survenue du cycle cardiaque suivant.

En revanche, dans le cas de la Figure 8b, l'activité du patient augmente, avec un niveau de variation du signal beaucoup plus important dans la durée de la fenêtre F3.

Le paramètre d'activité du patient est avantageusement évalué par redressement et intégration du signal S pendant la durée de la fenêtre F3, c'est-à-dire par sommation des valeurs absolues des échantillons de signal successivement recueillis pendant cette période.

La Figure 8c illustre un exemple où le niveau d'activité est encore plus élevé, correspondant typiquement à une situation d'exercice intense tel que course, montée d'escalier, etc. Il peut même arriver que dans ce cas le niveau moyen du signal S dans la fenêtre F3 de détection d'activité du patient soit supérieur au niveau moyen du signal en dehors de cette fenêtre, notamment dans des périodes correspondant à la fenêtre F1 de scrutation du signal à la recherche du premier pic de l'oscillation libre de l'ensemble pendulaire) et à la fenêtre F2 d'effacement de la phase de rebonds décrites en référence à la Figure 4.

Dans un tel cas, la fenêtre F3 ne peut plus être définie par les techniques exposées plus haut (détection du premier pic PSE puis application d'une fenêtre F2 d'effacement de la phase de rebonds), puisque les variations du signal électrique ne présentent plus l'allure qu'elles avaient auparavant, comme dans le cas des Figures 8a et 8b où il était possible de discriminer aisément le pic PSE caractérisant le début de l'oscillation libre de l'ensemble pendulaire. La fenêtre F3 ne pouvant plus être déterminée par analyse du signal pendant le cycle courant, elle peut être déterminée par exemple à partir de la valeur que cette fenêtre avait au(x) cycle(s) précédent(s), en conservant la même position temporelle par rapport à l'évènement cardiaque, et en conservant la même durée de fenêtre ou en ajustant cette durée d'un facteur prédéterminé (par exemple de 10 ms ou de 10 %) pour tenir compte du fait qu'une augmentation de l'activité s'accompagne très probablement d'une accélération du rythme cardiaque, dont il faudra tenir compte pour la définition de la fenêtre.

En variante, la durée de la fenêtre F3 peut être déterminée à partir du rythme cardiaque courant, par exemple sous forme d'un pourcentage de l'intervalle RR courant, éventuellement moyenné sur les quelques cycles précédents, cette fenêtre étant décalée d'une durée prédéterminée par rapport au marqueur de détection de la contraction ventriculaire (marqueur qui est donné par l'analyse du signal EGM, indépendante des perturbations mécaniques produites par l'activité intense du patient).

La Figure 9 est un organigramme 100 détaillant, selon un exemple illustratif de mise en œuvre de l'invention, la séquence d'opérations et de tests effectués sur le signal électrique S délivré par le module de récupération d'énergie en fonction des évènements cardiaques détectés, en vue de l'obtention de données qui permettront de déterminer un ou plusieurs paramètres physiologiques et/ou d'activité physique.

Le bloc 102 correspond à une étape préalable de remise à zéro des diverses mémoires et temporisations qui seront utilisées par la suite. Un paramètre W est également mis à zéro, ce paramètre indiquant la phase d'analyse dans laquelle se trouve l'algorithme au cours de ses diverses itérations successives, et peut prendre les valeurs suivantes :
- W = 0 :: valeur initiale, phase indéterminée,
- W = 1 :: phase de scrutation du signal électrique à la recherche du premier pic (pic PSE sur la Figure 4), correspondant à la fenêtre F1 de la Figure 4 et pendant laquelle le signal S produit des informations significatives pour l'obtention d'un paramètre physiologique,
- W = 2 :: phase d'effacement de la phase de rebonds, correspondant à la fenêtre F2 de la Figure 4, pendant laquelle le signal ne produit pas d'information significative pour l'obtention d'un paramètre physique ni physiologique,
- W = 3 :: phase postérieure à la phase de rebonds, correspondant à la fenêtre F3 de la Figure 4 et pendant laquelle le signal S produit des informations significatives pour l'obtention d'un paramètre d'activité physique.

L'algorithme est itéré régulièrement à chaque réception d'une impulsion de séquencement correspondant à une interruption délivrée par le microprocesseur (bloc 104).

Si un évènement cardiaque (typiquement un marqueur R ou V) est détecté, ou que la nature de la fenêtre n'est pas encore déterminée (W = 0), le test 106 dirige l'algorithme vers un ensemble d'étapes 108 et suivantes de gestion des évènements cardiaques.

Au bloc 108, un nouveau marqueur cardiaque est établi, et la mémoire des échantillons du signal S est remise à zéro.

S'il s'agit de la première itération (c'est-à-dire si W = 0, test 110), alors l'indicateur de fenêtre W est positionné à 1 et un compteur temporel est démarré (bloc 112) de manière à déclencher la scrutation du signal électrique à la recherche du pic de signal (pic PSE).

À chaque échantillon reçu du signal S (test 114), l'échantillon est mémorisé et les temps mesurés sont mis à jour (bloc 116). Si l'on est toujours en phase de recherche du pic de signal (W = 1, test 118), tant que le pic n'est pas atteint, c'est-à-dire tant que le niveau du signal S augmente, alors l'algorithme est itéré (test 120, retour à l'étape 104). Dans le cas contraire, l'instant de survenue et le niveau du pic PSE sont mémorisés (bloc 122) et, la phase de recherche du pic étant terminée, l'indicateur W est positionné à W = 2. Cette itération de l'organigramme est achevée avec retour à l'étape 104.

Pendant la phase de rebonds (W = 2, test 124), les échantillons de signal successifs sont analysés pour déterminer si cette phase de rebonds est terminée, selon les divers critères qui ont été explicités plus haut (niveau de signal au-dessous d'un seuil donné, écoulement d'un temps maximal, etc.). Si l'on se trouve toujours en phase de rebonds (test 126) l'algorithme est itéré (retour au bloc 104). Dans le cas contraire, l'indicateur W est positionné à W = 3 (bloc 128).

À chaque itération, l'algorithme recueillera un nouvel échantillon et le cumuler avec les précédents (bloc 130) pour permettre, comme expliqué plus haut, le redressement et l'intégration du signal sur toute la durée de la fenêtre de détection d'activité (W = 3).

Ces itérations se poursuivront jusqu'à détection d'un nouvel évènement cardiaque (test 106).

Ce nouvel évènement cardiaque, détecté par le test 132, déclenchera (bloc 134) diverses opérations de clôture de l'analyse du cycle, avec en particulier la mémorisation du cumul des échantillons obtenus précédemment au bloc 130, et déclenchera le comptage du temps pour déterminer l'instant de survenue du pic à venir du signal électrique (cette opération du bloc 134 étant la même que celle qui avait été opérée à l'initialisation au bloc 112). L'indicateur de fenêtre est alors positionné à W = 1 (bloc 136) pour indiquer que l'on se trouve à nouveau dans une fenêtre de scrutation du signal.

L'algorithme retourne alors à l'étape 114 d'attente d'un nouvel échantillon du signal S, qui sera traité de la même manière que décrit plus haut.

## Revendications

1. Un implant cardiaque autonome (10) de type capsule *leadless,* comprenant un corps d'implant (12) muni de moyens (18) d'ancrage à une paroi cardiaque, le corps d'implant logeant un ensemble électronique (14) et un module de récupération d'énergie avec un organe de stockage d'énergie (42) pour l'alimentation de l'ensemble électronique,
le module de récupération d'énergie étant apte à convertir en énergie électrique des sollicitations externes appliquées au corps de l'implant sous l'effet de mouvements d'une paroi à laquelle est ancré l'implant et/ou de variations de débit du flux sanguin dans le milieu environnant l'implant au rythme de battements cardiaques et/ou de vibrations de tissus cardiaques,
dans lequel le module de récupération d'énergie comprend :
- un ensemble inertiel (24, 28) soumis auxdites sollicitations externes ;
- un traducteur (24) apte à convertir l'énergie mécanique produite par les oscillations de l'ensemble inertiel en un signal électrique oscillant (S) ; et
- un circuit de gestion d'alimentation (40), apte à redresser et réguler ledit signal électrique oscillant (S), pour délivrer en sortie une tension ou un courant continus stabilisés pour l'alimentation dudit ensemble électronique (14) et/ou la recharge dudit organe de stockage d'énergie (42),
l'implant comprenant en outre un module d'analyse recevant en entrée le signal électrique (S) délivré par le traducteur et apte à analyser des variations de ce signal électrique oscillant (S) pour en déduire une valeur d'un paramètre physiologique du patient porteur de l'implant,
**caractérisé en ce qu'**il comprend en outre :
- un module de séquencement (46), comprenant :
• un circuit de détection d'événements cardiaques ventriculaires ou auriculaires successifs ; et
- un circuit de fenêtrage, apte à définir au moins une fenêtre temporelle prédéterminée (F1, F2, F3) entre deux évènements cardiaques consécutifs détectés,
et **en ce que** le module d'analyse est un module (44) apte à :
• analyser les variations instantanées dudit signal électrique oscillant (S) à l'intérieur de ladite au moins une fenêtre temporelle prédéterminée (F1, F2, F3) ainsi définie entre deux évènements cardiaques consécutifs détectés, et
• en déduire une valeur courante i) d'au moins un paramètre physiologique, et/ou ii) d'au moins un paramètre d'activité physique, du patient porteur de l'implant.

2. L'implant de la revendication 1, dans lequel l'ensemble inertiel comprend un ensemble pendulaire avec un élément élastiquement déformable (24) suivant au moins un degré de liberté, couplé à une masse inertielle (28).

3. L'implant de la revendication 2, dans lequel le module de récupération d'énergie comprend au moins une lame piézoélectrique (24) couplée à l'une de ses extrémités à la masse inertielle (28), ladite lame piézoélectrique formant à la fois ledit élément élastiquement déformable et ledit traducteur.

4. L'implant de la revendication 1, dans lequel le circuit de fenêtrage est apte à définir une première fenêtre temporelle (F1) de recherche d'un premier pic du signal électrique oscillant (S).

5. L'implant de la revendication 1, dans lequel le circuit de fenêtrage est apte à définir une deuxième fenêtre temporelle (F2) correspondant à une phase en oscillation libre amortie de l'ensemble inertiel.

6. L'implant de la revendication 5, dans lequel le circuit de fenêtrage est apte à rechercher un pic d'amplitude (PSE) de la première oscillation du signal électrique (S), et à définir la deuxième fenêtre temporelle (F2) en fonction de l'instant dudit pic d'amplitude (PSE).

7. L'implant de la revendication 4, dans lequel le module d'analyse est apte à déterminer une grandeur liée à au moins une partie de la durée d'une alternance de la première oscillation du signal électrique (S) ; et déduire au moins un paramètre physiologique du patient en fonction de ladite grandeur.

8. L'implant de la revendication 7, dans lequel ladite grandeur est l'une d'entre : l'amplitude (A_{PSE}) d'un premier pic de signal (PSE) de la première alternance du signal électrique oscillant (S) ; la dérivée (D_{PSE}) de l'amplitude du signal électrique oscillant (S) au début de la première alternance ; ou l'intégrale (I_{PSE}) de l'amplitude du signal électrique oscillant (S) sur tout ou partie de la durée de la première alternance.

9. L'implant de la revendication 4, dans lequel le module d'analyse est apte à : discriminer une première oscillation (PSE1) et une seconde oscillation (PSE2) consécutive du signal électrique survenant dans la première sous-fenêtre ; déterminer deux grandeurs liées à au moins une partie de la durée d'une alternance respective desdites première et seconde oscillations (PSE1, PSE2) ; et déduire i) deux paramètres physiologiques distincts du patient en fonction de ces deux grandeurs respectives ou ii) un paramètre physiologique du patient en fonction d'une combinaison de ces deux grandeurs respectives.

10. L'implant de la revendication 5, dans lequel le circuit de fenêtrage est apte à définir une troisième fenêtre temporelle (F3), postérieure à la deuxième fenêtre temporelle (F2), correspondant à une phase substantiellement non oscillante du système inertiel en oscillation libre amortie.

11. L'implant de la revendication 10, dans lequel le module d'analyse est apte à : déterminer une grandeur liée aux variations du signal électrique pendant la troisième fenêtre temporelle (F3) ; et déduire au moins un paramètre d'activité physique du patient en fonction de ladite grandeur.

12. L'implant de la revendication 11, dans lequel ladite grandeur est l'énergie du signal électrique redressé et intégré pendant la durée de la seconde sous-fenêtre.

## Patentansprüche

1. Autonomes Herzimplantat (10) vom Typ *leadless* Kapsel, umfassend einen Implantatkörper (12), der mit Mitteln (18) zur Verankerung an einer Herzwand versehen ist, wobei der Implantatkörper eine elektronische Anordnung (14) und ein Energierückgewinnungsmodul mit einem Energiespeicherorgan (42) für die Versorgung der elektronischen Anordnung aufnimmt,
wobei das Energierückgewinnungsmodul dazu eingerichtet ist, äußere Belastungen, die ausgeübt werden auf den Körper des Implantats unter der Einwirkung von Bewegungen einer Wand, an der das Implantat verankert ist, und/oder von Schwankungen der Durchflussmenge des Blutflusses in der Umgebung, die das Implantat umgibt, im Rhythmus von Herzschlägen und/oder von Vibrationen von Herzgewebe, in elektrische Energie umzuwandeln,
wobei das Energierückgewinnungsmodul umfasst:
- eine inertiale Anordnung (24, 28), die den äußeren Belastungen ausgesetzt ist;
- einen Wandler (24), der dazu eingerichtet ist, die von den Schwingungen der inertialen Anordnung erzeugte mechanische Energie in ein schwingendes elektrisches Signal (S) umzuwandeln; und
- eine Versorgungsverwaltungssteuerung (40), die dazu eingerichtet ist, das schwingende elektrische Signal (S) gleichzurichten und zu regeln, um am Ausgang eine stabilisierte Gleichspannung oder einen stabilisierten Gleichstrom für die Versorgung der inertialen Anordnung (14) und/oder das Wiederaufladen des Energiespeicherorgans (42) bereitzustellen,
wobei das Implantat ferner ein Analysemodul umfasst, das am Eingang das von dem Wandler bereitgestellte elektrische Signal (S) empfängt und dazu eingerichtet ist, Schwankungen dieses schwingenden elektrischen Signals (S) zu analysieren, um daraus einen Wert eines physiologischen Parameters des Patienten, der das Implantat trägt, abzuleiten,
**dadurch gekennzeichnet, dass** es ferner umfasst:
- ein Sequenzierungsmodul (46), umfassend:
• eine Schaltung für die Detektion von sukzessiven ventrikulären oder aurikulären Herz-Ereignissen; und
• eine Fensterschaltung, die dazu eingerichtet ist, wenigstens ein vorab bestimmtes Zeitfenster (F1, F2, F3) zwischen zwei detektierten aufeinander folgenden Herz-Ereignissen zu definieren,
und dass das Analysemodul ein Modul (44) ist, das eingerichtet ist zum:
• Analysieren der kurzzeitigen Schwankungen des schwingenden elektrischen Signals (S) im Inneren des wenigstens einen vorab bestimmten Zeitfensters (F1, F2, F3), das auf diese Weise zwischen zwei detektierten aufeinander folgenden Herz-Ereignissen definiert wurde, und
• daraus Ableiten eines aktuellen Wertes i) von wenigstens einem physiologischen Wert und/oder ii) von wenigstens einem Parameter der körperlichen Aktivität des Patienten, der das Implantat trägt.

2. Implantat nach Anspruch 1, wobei die inertiale Anordnung eine pendelartige Anordnung mit einem Element (24) umfasst, das gemäß wenigstens einem Freiheitsgrad elastisch verformbar ist und an eine inertiale Masse (28) gekoppelt ist.

3. Implantat nach Anspruch 2, wobei das Energierückgewinnungsmodul wenigstens eine piezoelektrische Zunge (24) umfasst, die an einem ihrer Enden an die inertiale Masse (28) gekoppelt ist, wobei die piezoelektrische Zunge gleichzeitig das elastisch verformbare Element und den Wandler bildet.

4. Implantat nach Anspruch 1, wobei die Fensterschaltung dazu eingerichtet ist, ein erstes Zeitfenster (F1) zum Suchen einer ersten Spitze des schwingenden elektrischen Signals (S) zu definieren.

5. Implantat nach Anspruch 1, wobei die Fensterschaltung dazu eingerichtet ist, ein zweites Zeitfenster (F2) zu definieren, das einer Phase in freier gedämpfter Schwingung der inertialen Anordnung entspricht.

6. Implantat nach Anspruch 5, wobei die Fensterschaltung dazu eingerichtet ist, eine Amplitudenspitze (PSE) der ersten Schwingung des elektrischen Signals (S) zu suchen und das zweite Zeitfenster (F2) in Abhängigkeit von dem Zeitpunkt der Amplitudenspitze (PSE) zu definieren.

7. Implantat nach Anspruch 4, wobei das Analysemodul dazu eingerichtet ist, eine Größe zu bestimmen, die mit wenigstens einem Teil der Dauer einer Halbperiode der ersten Schwingung des elektrischen Signals (S) verbunden ist; und wenigstens einen physiologischen Parameter des Patienten in Abhängigkeit von dieser Größe abzuleiten.

8. Implantat nach Anspruch 7, wobei die Größe eine ist aus: der Amplitude (A_{PSE}) einer ersten Signalspitze (PSE) der ersten Halbperiode des schwingenden elektrischen Signals (S); der Drift der Amplitude (D_{PSE}) des schwingenden elektrischen Signals (S) zu beginn der ersten halbperiode; oder dem Integral (D_{PSE}) der Amplitude des schwingenden elektrischen Signals (S) über die gesamte oder einen Teil der Dauer der ersten Halbperiode.

9. Implantat nach Anspruch 4, wobei das Analysemodul eingerichtet ist zum: Ausschließen einer ersten Schwingung (PSE1) und einer darauf folgenden zweiten Schwingung (PSE2) des elektrischen Signals, die in dem ersten Teil-Zeitfenster auftritt; Bestimmen von zwei Größen, die mit wenigstens einem Teil der Dauer einer jeweiligen Halbperiode der ersten und zweiten Schwingung (PSE1, PSE2) verbunden ist; und Ableiten i) von zwei verschiedenen physiologischen Parametern des Patienten in Abhängigkeit von diesen zwei jeweiligen Größen oder ii) von einem physiologischen Parameter des Patienten in Abhängigkeit von einer Kombination aus diesen zwei jeweiligen Größen.

10. Implantat nach Anspruch 5, wobei die Fensterschaltung dazu eingerichtet ist, ein drittes Zeitfenster (F3), das dem zweiten Zeitfenster (F2) zeitlich folgt, zu bestimmen, das einer im Wesentlichen nicht schwingenden Phase des inertialen Systems in freier gedämpfter Schwingung entspricht.

11. Implantat nach Anspruch 10, wobei das Analysemodul eingerichtet ist zum: Bestimmen von einer Größe, die mit den Schwankungen des elektrischen Signals während des dritten Zeitfensters (F3) verbunden ist; und Ableiten von wenigstens einem Parameter der körperlichen Aktivität in Abhängigkeit von der Größe.

12. Implantat nach Anspruch 11, wobei die Größe die Energie des elektrischen Signals ist, das während der Dauer des zweiten Teil-Fensters gleichgerichtet und integriert wird.

## Claims

1. An autonomous cardiac implant (10) of the leadless capsule type, having an implant body (12) provided with means (18) for anchoring to a cardiac wall, the implant body accommodating an electronic unit (14) and an energy harvesting module with an energy storage component (42) for powering the electronic unit,
the energy harvesting module being adapted to convert into electrical energy external stresses applied to the implant body under the effect of movements of a wall to which the implant is anchored and/or of blood flow rate variations in the environment surrounding the implant at the rhythm of heartbeats and/or of cardiac tissue vibrations,
wherein the energy harvesting module comprises:
- an inertial unit (24, 28) subjected to said external stresses;
- a translator (24) adapted to convert the mechanical energy produced by the oscillations of the inertial unit into an oscillating electrical signal (S); and
- a power management circuit (40), adapted to rectify and regulate said oscillating electrical signal (S), to output a stabilized direct voltage or current for powering said electronic unit (14) and/or for charging said energy storage component (42),
the implant further comprising an analysis module receiving as an input the electrical signal (S) provided by the translator and adapted to analyze variations of this oscillating electrical signal (S) to derive therefrom a value of a physiological parameter of the patient with the implant,
**characterized in that** it further comprises:
- a sequencing module (46), comprising:
a circuit for detecting successive ventricular or atrial cardiac events; and
a windowing circuit, adapted to define at least one predetermined time window (F1, F2, F3) between two consecutive detected cardiac events;
and **in that** the analysis module is a module (44) adapted to:
analyze the instantaneous variations of said oscillating electrical signal (S) inside said at least one predetermined time window (F1, F2, F3) so defined between two consecutive detected cardiac events, and
derive therefrom a current value i) of at least one physiological parameter, and/or ii) at least one physical activity parameter, of the patient with the implant.

2. The implant of claim 1, wherein the inertial unit comprises a pendular unit with an element (24), elastically deformable according to at least one degree of freedom, coupled to an inertial mass (28).

3. The implant of claim 2, wherein the energy harvesting module comprises at least one piezoelectric beam (24) coupled at one of its ends to the inertial mass (28), said piezoelectric beam forming both said elastically deformable element and said translator.

4. The implant of claim 1, wherein the windowing circuit is adapted to define a first time window (F1) for searching for a first peak of the oscillating electrical signal (S).

5. The implant of claim 1, wherein the windowing circuit is adapted to define a second time window (F2) corresponding to a damped free oscillation phase of the inertial unit.

6. The implant of claim 5, wherein the windowing circuit is adapted to search for a peak of amplitude (PSE) of the first oscillation of the electrical signal (S), and define the second time window (F2) as a function of the moment of said peak of amplitude (PSE).

7. The implant of claim 4, wherein the analysis module is adapted to determine a quantity linked to at least a part of the duration of an alternation of the first oscillation of the electrical signal (S); and derive at least one physiological parameter of the patient as a function of said quantity.

8. The implant of claim 7, wherein said quantity is one between: the amplitude (A_{PSE}) of a first signal peak (PSE) of the first alternation of the oscillating electrical signal (S); the derivative (D_{PSE}) of the amplitude of the oscillating electrical signal (S) at the beginning of the first alternation; or the integral (I_{PSE}) of the amplitude of the oscillating electrical signal (S) over all or part of the duration of the first alternation.

9. The implant of claim 4, wherein the analysis module is adapted to: discriminate a first oscillation (PSE1) and a second, consecutive oscillation (PSE2) of the electrical signal occurring in the first sub-window; determine two quantities linked to at least a part of the duration of a respective alternation of said first and second oscillations (PSE1, PSE2); and derive i) two distinct physiological parameters of the patient as a function of these two respective quantities or ii) a physiological parameter of the patient as a function of a combination of these two respective quantities.

10. The implant of claim 5, wherein the windowing circuit is adapted to define a third time window (F3), posterior to the second time window (F2), corresponding to a substantially non-oscillating phase of the inertial system in damped free oscillation.

11. The implant of claim 10, wherein the analysis module is adapted to: determine a quantity linked to the variations of the electrical signal during the third time window (F3); and derive at least one physical activity parameter of the patient as a function said quantity.

12. The implant of claim 11, wherein said quantity is the energy of the electrical signal, rectified and integrated, for the duration of the second sub-window.
